Europäisches Patentamt

European Patent Office

Office européen des brevets

(11) Publication number: **0 143 850**
**B1**

(12) **EUROPEAN PATENT SPECIFICATION**

(45) Date of publication of patent specification: **28.02.90**

(21) Application number: **84901399.0**

(22) Date of filing: **29.03.84**

(86) International application number:
**PCT/JP84/00147**

(87) International publication number:
**WO 84/03887 11.10.84 Gazette 84/24**

(51) Int. Cl.⁵: **C 07 K 15/04, C 12 P 21/00,
A 61 K 37/02** // (C12P21/00,
C12R1:91)

(54) **NOVEL PHYSIOLOGICALLY ACTIVE PROTEINACEOUS SUBSTANCE.**

(30) Priority: **31.03.83 JP 53860/83**

(43) Date of publication of application:
**12.06.85 Bulletin 85/24**

(45) Publication of the grant of the patent:
**28.02.90 Bulletin 90/09**

(84) Designated Contracting States:
**BE CH DE FR GB LI NL SE**

(56) References cited:
THE JOURNAL OF IMMUNOLOGY, vol. 128, no.
6, June 1982, pages 2393-2398, The American
Association of Immunologists, Baltimore, US;
J.C. LEE et al.: "Constitutive production of a
unique lymphokine (IL 3) by the WEHI-3 cell line"

CHEMICAL ABSTRACTS, vol. 100, no. 13, 26th
March 1984, page 140, abstract 97366t,
Columbus, Ohio, US; F. TAKETAZU et al.:
"Clonal growth of human acute myeloid
leukemia cells (ML-1 and HL-60) in serum-free
agar medium", & CANCER RES. 1984, 44(2),
531-5

(73) Proprietor: **KYOWA HAKKO KOGYO CO., LTD.**
**Ohtemachi Bldg., 6-1 Ohtemachi I-chome**
**Chiyoda-ku Tokyo 100 (JP)**
(73) Proprietor: **OKABE, Tetsuro**
**5-11-15-306, Koishikawa**
**Bunkyo-ku Tokyo 112 (JP)**

(72) Inventor: **SATO, Seiji**
**1511-7, Okada Atsugi-shi**
**Kanagawa 243 (JP)**
Inventor: **OGATA, Tomoko**
**6-24-12, Hiyoshidai Tomisato-mura**
**Inba-gun Chiba 286-02 (JP)**
Inventor: **FUJIWARA, Kazumi**
**3-6-6, Asahi-machi Machida-shi**
**Tokyo 194 (JP)**
Inventor: **FUJIYOSHI, Nobuo**
**1-12-2, Asahi-machi Machida-shi**
**Tokyo 194 (JP)**
Inventor: **OKABE, Tetsuro**
**5-11-15-306, Koishikawa, Bunkyo-ku**
**Tokyo 112 (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

The Biochemical Journal, Vol. 148, (1975),
Gerald B. Price et al "The Isolation and Properties
of Granulocytic Colony - Stimulating Activities
from Medium Conditioned by Human Peripheral
Leucocytes" P. 209-217

## Description

The present invention relates to novel physiologically active proteinaceous substances having an activity of promoting DNA synthesis, and to a process for producing the said substances by culturing human myelogenic leukemia cells.

Heretofore, the following examples have been known as substances having an activity of promoting DNA synthesis.

(1) PDGF (Platelet-derived growth factor)
Proc. Natl. Acad. Sci., 76, 1809 (1979)

(2) TGF (Transforming growth factor)
J. Biol. Chem. 257, 5220 (1982)

(3) EGF (Epidermal growth factor)
Anal. Biochem. 111, 195 (1981)

(4) FGF (Fibroblast growth factor)
Ann. Rev. Biochem. 45, 531 (1976)

(5) FSLA (Fibroplast Somatomedin like activity)
J. Cellular Physiology 107, 317 (1981)

(6) IGF—I (Insulin like growth factor)
J. Endocr. 85, 267 (1980)

(7) IGF—II (Insulin like growth factor)
Diabetes 31, 2823 (1982)

(8) MSA (Multiplication stimulating activity)
Nature 272 (23), 776 (1978)

These substances having an activity of promoting DNA synthesis are produced using media containing proteinaceous substances such as serum, and thus it is difficult to identify the substances as those produced by cultured cells and to separate the substances from the medium components. Owing to these difficulties, these substances have not yet a practical significance.

The present inventors have found that, when human myelogenic leukemia cells are cultured in a protein-free medium, the culture supernatant contains substances which remarkably promote uptake $^3$H-thymidine into cells and promote DNA synthesis in an uptake test system using mouse fibroplast strain 3T3 and chick embryo fibroplast, and have established the present invention.

The present invention provides physiologically active proteinaceous substances exhibiting an activity of promoting DNA synthesis which are produced by a myelogenic leukemia cell strain and which are obtainable by culturing a myelogenic leukemia cell strain in a protein-free medium accumulating physiologically active proteins exhibiting an activity of promoting DNA synthesis in the culture broth, and recovering the proteins from the culture broth.

The substances of the present invention can be classified into two groups, i.e., the one having a molecular weight of 6,300 ± 500 and the one having a molecular weight of 9,000—13,000, and can be further classified into the following species according to isoelectric points.

EP 0 143 850 B1

TABLE 1

| | No. | Molecular weight | Isoelectric point (pI) |
|---|---|---|---|
| | 1 | 9,200 ± 500 | 8.55 ± 0.1 |
| | 2 | 10,000 ± 500 | 6.9 ± 0.1 |
| | 3 | 9,400 ± 500 | 8.25 ± 0.1 |
| | 4 | 10,000 ± 500 | 6.7 ± 0.1 |
| I | 5 | 10,500 ± 500 | 5.8 ± 0.1 |
| | 6 | 10,500 ± 500 | 5.6 ± 0.1 |
| | 7 | 10,500 ± 500 | 6.09 ± 0.1 |
| | 8 | 11,000 ± 500 | 4.7 ± 0.1 |
| | 9 | 11,800 ± 500 | 4.19 ± 0.1 |
| | 10 | 12,800 ± 500 | 3.54 ± 0.1 |
| | 11 | 6,300 ± 500 | 8.0 ± 0.1 |
| | 12 | 6,300 ± 500 | 8.4 ± 0.1 |
| II | 13 | 6,300 ± 500 | 8.5 ± 0.1 |
| | 14 | 6,300 ± 500 | 9.0 ± 0.3 |
| | 15 | 6,300 ± 500 | 9.6 ± 0.3 |

The substances of the present invention are not decomposed by deoxyribonuclease, ribonuclease and neuraminidase, and are inactivated by proteinase such as trypsin and chymotrypsin. Thus, they can be regarded as proteinaceous or peptidic substances.

The substances of the present invention have an activity of promoting uptake of $^3$H-thymidine into chick embryo fibroblasts and mouse fibroblast strain 3T3 and promoting DNA synthesis. Furthermore, the substances of the present invention also promote propagation of mouse fibroblast strain 3T3.

Molecular weights and isoelectric points of said well known active substances are given below for comparison.

4

| Substance | Molecular weight | Isoelectric point (pI) |
|---|---|---|
| PDGF | 13,000—16,000 | 9.8 |
| TGF | 7,400 | — |
| EGF | 6,000 | 4.5 |
| FGF | 13,000 | 9.7 |
| FSLA | 29,000 | 4.7 |
|  | 16,500 | 6.1—6.3 |
| IGF—I | 7,649 | 8.3 |
| IGF—II | 7,500 | 6.5 |
| MSA | 40,000 | — |
|  | 20,000 | — |
|  | 11,000 | — |
|  | 7,000 | — |

It is evident that the substances of the present invention are novel substances because none of them have the same molecular weight or isoelectric point as these known substances and also they have a different origin.

The substances of the present invention are formed and accumulated mainly in a culture supernatant by culturing human myelogenic leukemia cells in a medium, and thus can be recovered therefrom.

Any of human myelogenic leukemia cells can be used so long as they can produce the substances of the present invention, and K562—T1 strain can be mentioned as a suitable strain. The K562—T1 strain consists of cells adapted to a protein-free medium, which are obtained by culturing K562 strain in Ham F10 medium initially containing 10% fetal calf serum while lowering the serum concentration successively for an adaptation period of about one year. The K562 strain is a well known cell strain disclosed in Proc. Natl. Acad. Sci., USA., 76, 1293 (1979), Blood, 45, 321 (1975), GANN 73, 97 (1982), and is generaly available.

As the medium, protein-free media such as Ham F10 medium and Ham F12 medium (products of Flow Laboratory Inc.), Dulbecco MEM medium, MEM medium and RPMI—1640 medium (products of Nissui Seiyaku Co., Ltd.), and their mixed media can be employed. Appropriate amounts of glutamine (0.5—5 mM/ml), antibiotics [penicillin (25 U/ml), streptomycin (25 µg/ml), etc], sodium bicarbonate (0.01%), etc. may be added to the medium if necessary.

For the culturing, various culturing bottles, Petri dishes, roller bottles, spinner flasks, jar fermenters, can be employed. Culturing is usually carried out at a seed cell density of $5 \times 10^4 — 1 \times 10^6$ cells/ml and 30—40°C for 2—4 hours whereby the substances of the present invention are formed mainly in a culture broth according to the respective cell density. For example, by culturing at the seed cell density $1 \times 10^5$ cells/ml and 37°C for 2 days, 300 unit/ml active substance is formed in the culture broth.

The substances of the present invention are recovered from the culture broth in the following manner. That is, the supernatant of the resulting culture broth is concentrated by freeze-drying, ultrafiltration, strongly acidic ion exchange resin. Elution is carried out with a weakly basic buffer or an alkali solution of low concentration. Furthermore, low molecular substances of medium origin are removed by gel filtration. As the gel filtering agent, Sephadex® (made by Pharmacia Fine Chemicals, Inc.), Biogel® (made by Bio-Rad Laboratories, Inc.), Control Pore Glass® (made by Corning Glass Works, Inc.), Toyopearl® (made by Toyo Soda Co., Ltd.), can be employed. Since the substances of the present invention have a high molecular weight, many low molecular substances have an influence upon the biological activity assays can be removed by restricting the molecular weight-fractionating range to 1,000—5,000.

By this operation, the active fractions can be fractionated substantially at the void volume position.

The recovery of the active fractions is about 80%, and when only the measurement value at 280 nm is calculated and converted to the absorption at 280 nm of bovine albumin among the three wavelength measurements at 280 nm, 260 nm and 340 nm, the specific activity is increased about 200-fold. These active fractions are joined together and concentrated by freeze-drying.

Then, the active fractions are fractionated according to the molecular weights. The active fractions in an amount of 1/100 of the gel amount are passed through a column of Biogel or Sephadex, and then elution is carried out with 0.05M phosphate buffer as an eluting solution at a flow rate of 0.1 in S.V.

The active fractions are separated according to the respective molecular weights, and fractionated into three groups. The groups are designated as Fractions I, II and III in the order of molecular weight, I being the highest, and the ratio of I:II:III becomes 7:4:3. The entire activity recovery shows a substantial quantitative recovery in this step and the specific activity is increased 720-fold.

For isoelectric point fractionation, these active fractions are desalted with strongly basic ion exchange resin such as Dowex® 50W × 8 (made by Dow Chemical Co., Inc.), Amberlite® IR—120 (made by Bio-Rad Laboratory, Inc.), SK102 (made by Mitsubishi Kasei Kogyo Co., Ltd.), SP-Sephadex C—25 (made by Pharmacia Fine Chemicals, Inc.). After adsorption at pH 3—7, the column is washed with distilled water and eluted with 0—0.3N aqueous ammonia. The recovery in this step is 30—50%, and the activity ratio of the fractions is I:II:III = 7:2:3.

Isoelectric point electrophoresis is carried out at 900V and 10 mA for 48 hours in a column with 100 ml of LKB8100 (made by LKB, Sweden) at an ampholite concentration of 1.9—0.625% and at a pH gradient of 3—10 while cooling to 4°C. After the completion of electrophoresis, fractionation is carried out using a fraction collector, and pH and activity of the respective fractions are measured.

In this step, 5 species of active fractions shown in the said group I can be obtained from Fraction I, and 6 species of active fractions shown in the said group II can be obtained from Fraction II, while no characteristic active species are obtained from Fraction III. The ampholites are removed from the respective fractions through a PD—10 column (G—25 column made by Pharmacia Fine Chemicals, Inc.). In this step, the recovery of 0.5—2% as the ultimate recovery, and the specific activity is increased 3,600 to 14,400-fold.

Procedures for determining biological activities of the present invention are described below.

1. Evaluation of ability to promote DNA synthesis:

A) Evaluation system using 3T3—L1 cells:

Evaluation is carried out with a microplate with 24 holes (made by Nunc. Inc., Denmark) as a culturing vessel, a Dulbecco, et. al. — modified Eagle medium (DME medium, made by Nissui Seiyaku Co., Ltd.) containing glutamine (0.29 mg/ml), sodium bicarbonate (0.1%), penicillin 15 units/ml), streptomycin (15 µg/ml) and heat-inactivated fetal calf serum (made by Flow Laboratory, Inc.) (medium containing 10% serum is hereinafter referred to as liquid I and that containing 0.4% serum as liquid II) and 3T3—L1 cells (ATCC—92, available from Dai-Nippon Seiyaku Co., Ltd.).

A cell suspension ($2 \times 10^4$ cells/ml) is cultured in liquid I in air containing 5% carbon dioxide at 37°C for 5 hours, and then the liquid I is exchanged with liquid II, followed by culturing for 24 hours. Then, 0.1 ml of a sample is added, followed by culturing for 16 hours. $^3$H-thymidine is added and culturing is carried out for one hour. The amount of $^3$H-thymidine incorporated into DNA is measured with a liquid scintillation counter (made by Aroca, Inc.)

B) Evaluation system using CEF (chick embryo fibroblast cells:

Evaluation is carried out with the same culturing vessel as used in A), a Ham F10 medium (made by Flow Laboratory, Inc.) containing glutamine (0.29 mg/ml), sodium bicarbonate (0.1%), penicillin (15 units/ml), streptomycin (15 µg/ml) and 0.4% heat-inactivated fetal calf serum (liquid III), an MEM medium containing glutamine (0.29 mg/ml) and sodium bicarbonate (0.1%) (liquid IV) and 2nd generation CEF cells (obtained by incubating fertilized hen's eggs for 12 days to prepare first generation cells and then prepare the second generation after 3 days).

A cell suspension ($3.3 \times 10^5$ cells/ml) is cultured in liquid III in air containing 5% carbon dioxide at 37°C for 72 hours. Then, the liquid III is exchanged with liquid IV, and 0.1 ml of a sample is added thereto, followed by the same operations as in A).

C) Titer designation:

Titer is calculated according to the procedure disclosed in Biochemistry *4* (2), 169 (1972), wherein an ability to incorporate $^3$H-thymidine into DNA shown by 100 ng/ml insulin is designated as 1,000 units.

2. Procedure for determining an activity of promoting cell propagation with mouse 3T3 cells:

Determination is carried out with a microplate with 24 holes (made by Nuno, Inc., Denmark), a Dulbecco, et. al. — modified Eagle medium (DME medium, made by Nissui Seiyaku Co., Ltd.) containing glutamine (0.29 mg/ml), sodium bicarbonate (0.1%), penicillin 15 units/ml) streptomycin (15 µg/ml) and heat-inactivated fetal calf serum (medium containing 10% serum is hereinafter referred to as liquid A and that containing no serum as liquid B), and 3T3—L1 cells.

A cell suspension ($3 \times 10^4$ cells/ml) is cultured in liquid A in air containing 5% carbon dioxide at 37°C for 4 hours. Then, the medium is exchanged with liquid B, and 0.1 ml of a sample is added thereto. After culturing for 48 hours, the number of cells is counted.

The substances of the present invention having an action of promoting DNA synthesis on a broad range of cells have the following actions and various uses also.

1) They promote propagation of a cell system having an ability to produce antibodies, and can be used as an immune activator.

2) They promote propagation of a cells or erythrocyte system and thus can be applied to anemia.

6

3) They promote propagation of granulocytes and macrophages, and an anti-cancer action can be expected owing to prevention of infection and immune-activating action.

4) They promote propagation of a fibroblasts and thus can be applied as a curing agent for wounds.

5) They promote propagation of a cells which produce tumor necrosis factor considered to have an anti-cancer action, and thus, an effect can be expected as a factor promoting propagation of cells of macrophage system or as a factor promoting propagation of fibroblasts, cells of lymphoblast system which produces interferon, by addition to a medium free from serum.

These functions and effects are shown in Examples 2 and 3.

Fig. 1 shows an elution pattern of the substances of the present invention in Example 1 (1), wherein the activity refers to an activity of promoting the uptake of $^3$H-thymidine (the same shall apply hereinafter).

Fig. 2 shows an elution pattern of the substance of the present invention in Example 1 (2).

Fig. 3 shows an isoelectric point electrophoresis diagram of I.

Fig. 4 shows an isoelectric point electrophoresis diagram of II.

Fig. 5 shows an elution pattern of the substance of the present invention in relation to the molecular weight markers.

Fig. 6 shows a relationship between the molecular weight and the eluting position.

Fig. 7 is an elution pattern of the substance of the present invention in relation to the molecular weight markers.

Example 1

(1) Myelogenic leukemia cell K562—T1 strain was subjected to stationary culture as seed cells at 37°C for 4 days, and a cell supernatant was recovered. As the medium, Ham F10 medium (protein-free medium) containing glutamine (4 mM), penicillin (25 U/ml), streptomycin (25 μg/ml), and sodium bicarbonate (0.01%) was used.

The recovered cell supernatant (3,000 ml) was subjected to centrifugation (6,000 rpm for 10 minutes), and the supernatant (3,000 ml) was freeze-dried.

Dried powder (about 30 g) was dissolved in distilled water (43 ml) to make the concentration 70 times that of the cell supernatant. The solution was subjected to gel filtration with Biogel P—6 (made by Bio-Rad Laboratory, Inc.). The amount of the sample was 1/20 (W/V) of the gel amount, and elution was carried out with 0.05M phosphate buffer (pH 7.0) at a flow rate of 0.1 in S.V. The eluate was recovered in 10 ml fractions.

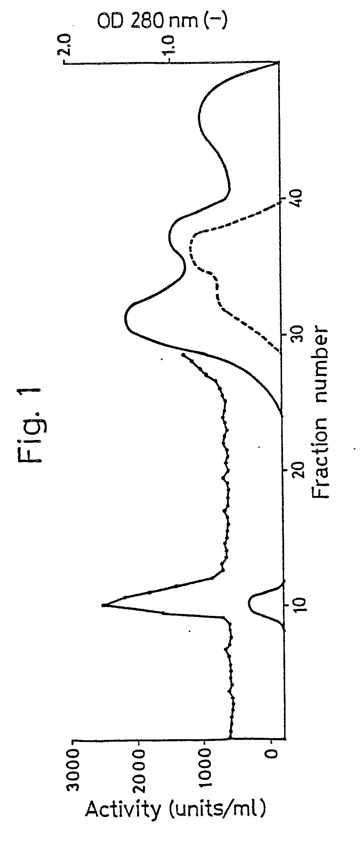

Elution pattern is shown in Fig. 1.

Low molecular substances were substantially completely removed, and an activity of promoting the uptake of $^3$H-thymidine appeared around the void volume (V0). The active fractions had also an effect of promoting the propagation of mouse 3T3—L1 cells, and thereby the number of cells propagated became 2.3 times as many as that in the case where no sample was added.

The recovery was 80%, and the specific activity was increased 210-fold, when the absorption of 280 nm was used to obtain the protein content in terms of bovine albumin amount among three wavelength absorbances at 260 nm, 280 nm and 340 nm, using Shimadzu Spectrophotometer UV240.

(2) Then, 60 ml of the said active fractions was collected, freeze-dried, and further fractionated likewise with Biogel P—60 (made by Bio-Rad Laboratory, Inc.). That is, 140 mg of the sample was dissolved in 4 ml of distilled water, and the solution was passed through a 100-ml column packed with Biogel P—60. Elution was carried out with 0.05M phosphate buffer (pH 7.0) at a flow rate of 0.1 in S.V. The eluate was recovered in 3 ml fractions.

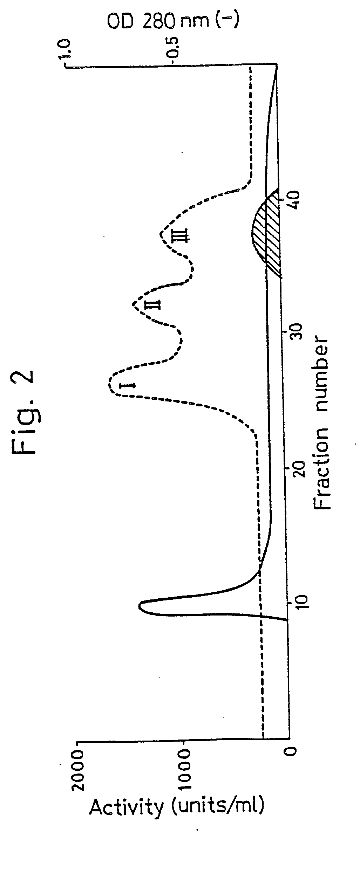

Elution pattern is shown in Fig. 2.

Active fractions were separated into three groups, and can be separated from high molecular impurities around V0. The three active groups were substantially quantitatively eluted together, and the specific activity was increased 720-fold. The groups were designated as I, II and III, respectively, in the order of molecular weight, I being the highest, and the activity ratio of these active fractions was 7:4:3, while their specific activities were substantially equal to one another.

(3) The active fractions I, II and III, (24 ml, 12 ml and 12 ml, respectively) were passed through a column packed with 1 ml of a strongly acidic ion exchange resin, Dowex 50W × 8 (Na$^+$ form, made by Dow Chemical Co.), and the column was washed in 15 ml of distilled water. Elution was carried out with 25 ml of distilled water and 25 ml of a 3N aqueous ammonia according to the linear concentration gradient method.

By this operation, salts were substantially completely removed. After the elution, the active fractions were freed from ammonia by freeze-drying.

|  | I | II | III |
| --- | --- | --- | --- |
| Recovery | 20% | 9% | 14% |
| Specific activity | 3,600 | 14,400 | 5,000 |

(4) Isoelectric points of the above fractions were determined using an 8100—1 column (made by LKB) with 1.9—0.625% of ampholite at pH 3—10. As an anolyte at the bottom of 110 ml-column was employed

EP 0 143 850 B1

1.0 m phosphoric acid, and as a catholyte at the top was employed 1% ethylenediamine, and a concentration gradient of 50%—5% was made by a gradient mixer. Electrophoresis was conducted at 900 volts and 4°C for 48 hours, and after the separation, the sample was fractionated into a fraction collector in 1 ml portions to measure pH. Biological activity was measured after separation of ampholite using PD—10 column (G—25 column made by Pharmacia Fine Chemicals, Inc.).

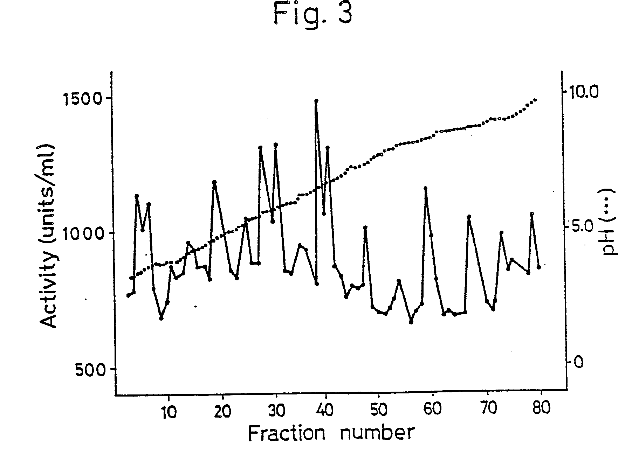

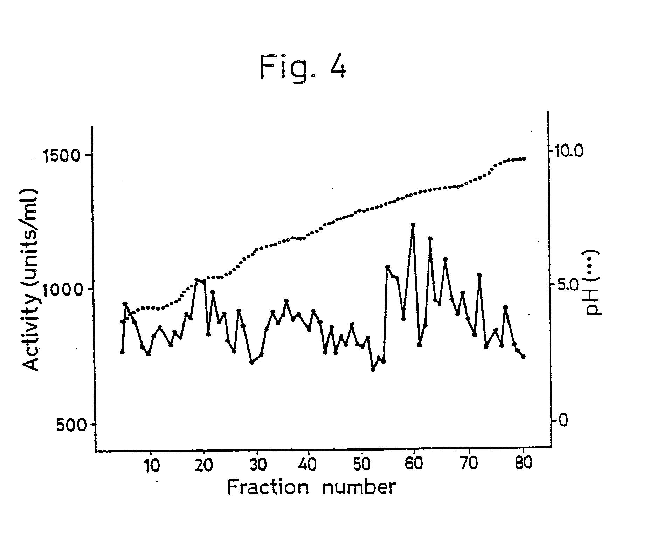

Isoelectric point electrophoresis diagrams of I and II are shown in Figs. 3 and 4, respectively. The result of I showed pI values of 8.55, 6.9, 8.25, 6.7, 5.8, 5.6, 6.09, 4.7, 4.19 and 3.54, and the result on II showed pI values of 8.0, 8.4, 8.5, 9.0 and 9.6. Thus, it seems that I and II are mixtures of physiologically active proteinaceous substances having similar molecular weights and different amino acid compositions.

No characteristic peak was observed in the case of III.

(5) The active fractions obtained in (2) above were freeze-dried, and then dissolved in 0.05M phosphate buffer (pH 7.0) in a concentration of 15,000 U/ml. The solution was passed through a column of 1.5 cm inner diameter packed with 100 ml of Sephadex G—50 (made by Pharmacia Fine Chemicals, Inc.). Elution was carried out with 0.05M phosphate buffer (pH 7.0) at a flow rate of 0.05 in S.V.

To obtain V0 values, 3 mg/ml γ-globulin (molecular weight: 160,000), 3 mg/ml cytochrome C (molecular weight: 12,500, 3 mg/ml insulin (molecular weight: 5,700) and 3 mg/ml bacitracin (molecular weight: 1,450) were used as molecular weight markers. Since insulin has a low solubility under the neutral condition, it was eluted with 0.1N acetic acid. The eluting position of each marker was determined from the absorbancy at 280 nm.

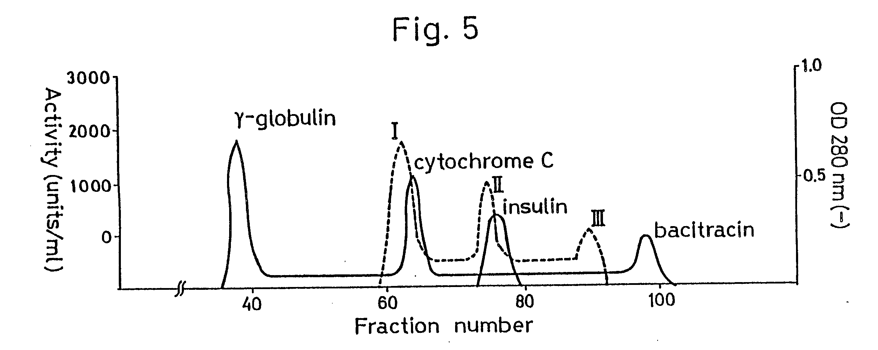

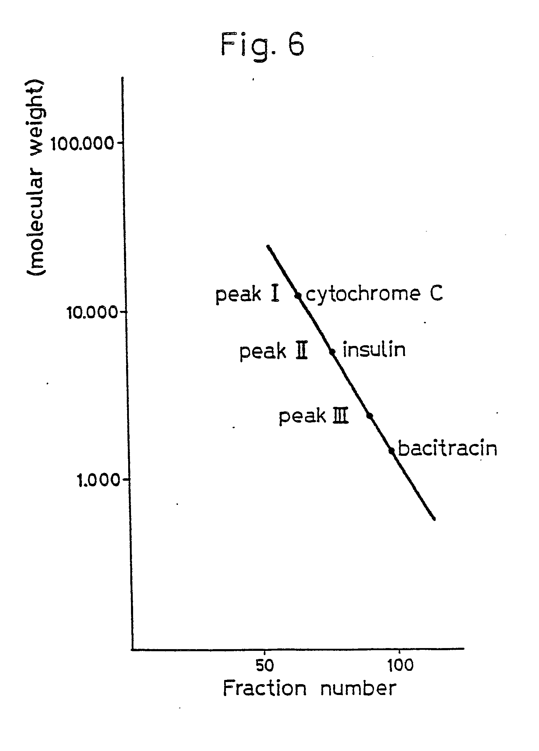

The results are shown in Fig. 5. To determine the molecular weight from the eluting positions, a relationship between the molecular weights and the eluting positions is shown in Fig. 6. These markers are positioned on a straight line on a semilogarithmic graph.

Active fraction I showed molecular weights of 9,000—13,000, II 6,300 ± 500, and III 2,400 ± 500.

It is seen from the foregoing results that the active fractions contain at least 15 species of substances shown in Table 1.

(6) To determine the respective molecular weights, active fraction I obtained in (5) above was subjected to gel filtration by high pressure liquid chromatography. The high pressure liquid chromatography was carried out using BECKMAN HPLC 342 (made by Beckman, Inc.), and as the gel filtration column, TSK—GPC column G—3,000SW (7.5φ × 600 cm) (made by Toyo Soda Kogyo Co., Ltd.). As the solvent, a mixture of 0.15M sodium chloride and 0.02M sodium acetate in the ratio of 1:1 was used at pH 6.0 as the molecular weight markers, 0.1 mg/0.2 ml cytochrome C (molecular weight: 12,500), 0.1 mg/0.2 ml trypsin inhibitor (molecular weight: 21,500), and 0.1 mg/0.2 ml aprotinin (molecular weight: 6,500) were used. The markers were positioned on a straight line on a logarithmic graph from their eluting positions. The components of the fraction were respectively subjected to gel filtration, and it was found that those appearing on the side of higher molecular weight by Sephadex G—50 had the following molecular weights.

| | No. | Molecular weight | Isoelectric point (pI) |
|---|---|---|---|
| | 1 | 9,200 ± 500 | 8.55 ± 0.1 |
| | 2 | 10,000 ± 500 | 6.9 ± 0.1 |
| | 3 | 9,400 ± 500 | 8.25 ± 0.1 |
| | 4 | 10,000 ± 500 | 6.7 ± 0.1 |
| I | 5 | 10,500 ± 500 | 5.8 ± 0.1 |
| | 6 | 10,500 ± 500 | 5.6 ± 0.1 |
| | 7 | 10,500 ± 500 | 6.09 ± 0.1 |
| | 8 | 11,000 ± 500 | 4.7 ± 0.1 |
| | 9 | 11,800 ± 500 | 4.19 ± 0.1 |
| | 10 | 12,800 ± 500 | 3.54 ± 0.1 |

(7) Molecular weight of the components of the active fraction II obtained in (5) above having pI values of 8.4, 9.0 and 9.6 were determined by high pressure liquid chromatography. Analysis was made using HSG—30W column for gel filtration and Shimadzu HPLC, LC—4A. Detection was made with UV at 280 nm, and 29 μl of the sample was injected. As the eluting solution, a buffer of 0.1M $KH_2PO_4$, 0.1M $Na_2HPO_4$ and

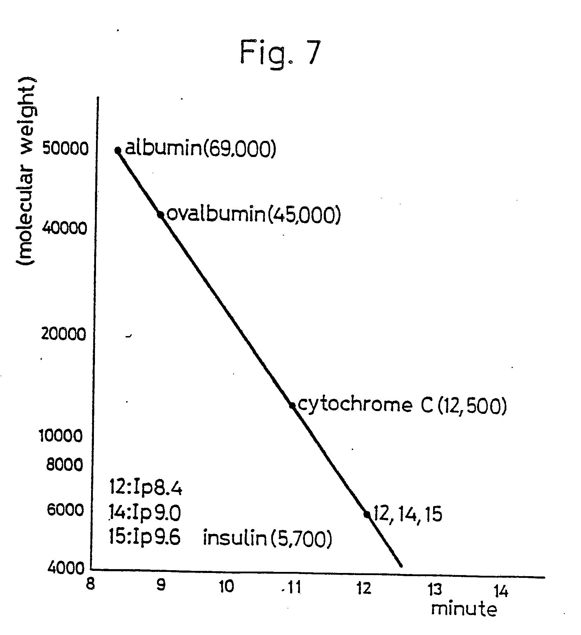

0.1M $Na_2SO_4$ (pH 6.8) was passed at a rate of 0.7 ml/min. As the molecular weight markers, 0.2 mg/ml each of albumin (molecular weight: 69,000, ovalbumin (molecular weight: 45,000), cytochrome C (molecualar weight: 12,500) and insulin (molecular weight: 5,700) were used, and the calibration curve shown in Fig. 7 was obtained.

It was found that each component had a molecular weight of 6,000.

Example 2

Action of promoting DNA synthesis on human lymphoblast cells: Active fraction I obtained in Example 1 was examined on the activity of promoting DNA synthesis of Namalva strain of the lymphoblast cells derived from Burkitts lymphona.

The Namalva strain was cultured at 37°C in a medium prepared by adding glutamine (4 mM), streptomycin (25 µg/ml), penicillin (25 U/ml), HEPES (10 mM), sodium bicarbonate (0.01%) and fetal calf serum (10%) to RPMI—1640 medium made by Nissui Seiyaku Co., Ltd. The cells were collected by centrifugation, and suspended in the said medium freed from the fetal calf serum at a concentration of $5 \times 10^5$ cells/ml. The suspension was poured in portions into a multidish plate with 24 holes and cultured at 37°C. After 3 days, the medium was exchanged with a fresh medium, followed by culturing at 37°C for 2 days. Then, the medium was exchanged again with a fresh medium, and samples (dilutions of active fraction I) and comparative samples (FCS and insulin) were added thereto. After culturing at 37°C for 6 hours, tritium thymidine was added thereto to $1.85 \times 10^4$ Bq/well (0.5 µCi/well), and the activities of its uptake were measured according to the ordinary procedure. The measurement values are averages of two runs of each test.

| | Sample dilution | | | | Comparative sample | | |
|---|---|---|---|---|---|---|---|
| 3H—TdR | ×2 | ×4 | ×8 | ×16 | FCS 0.5% | Insulin 100 ng/ml | 0 |
| Uptake cpm | 365 | 305 | 153 | 260 | 338 | 431 | 194 |

Example 3

Activity of promoting DNA synthesis on human leukemia cells:

Active fraction I obtained in Example 1 was examined on the ability to promote DNA synthesis of various leukemia cells.

K562 strain is an immature leukemia cell strain which is considered to be differentiable into an erythrocytic system and has been disclosed in Blood *45*, 321 (1975) as a human myelogenic leukemia cell strain. ML—1 strain has been established as an acute myelogenic leukemia (myeloblast) cell strain in Cancer Res. *42* 5152—5158 (1982) and is considered to be differentiable into macrophage monocytes. U—937 strain is a human hystocytic leukemia cell strain registered as ATCC CRL1593 and is a monocyte-like cell strain. THP—1 strain is a strain disclosed in Int. J. Cancer *26* 171—176 (1982) as human acute monocytic leukemia cell strain and is considered to be differentiable into macrophage.

An activity of promoting DNA synthesis on these cell strains which are considered to be in a series of differentiating steps was measured in the following manner.

Each of these cell strains was cultured at 37°C in a medium prepared by adding glutamine (4 mM), streptomycin (25 µg/ml), penicillin (25 U/ml), HEPES (10 mM) and sodium bicarbonate (0.01%), and further fetal calf serum (5%) to RPMI—1640 medium made by Nissui Seiyaku Co., Ltd. The cells were suspended in the said serum free medium. The suspension was poured in portions into a microtiter plate with 96 holes to $1 \times 10^4$ cells/well.

The suspensions of ML—1 and U—937 were cultured for 24 hours and those of K562 and THP—1 were cultured for 3 days. Then, 10 µl each of samples of various concentrations were added to the titer plates and culturing was conducted for 16—20 hours. Then, $3.7 \times 10^3$ Bq (0.1 µCi) of tritium thymidine was added to conduct pulse labelling for 8 hours. After the labelling, the cells were collected onto a filter paper with an autoharvester, and the filter was washed with 3 ml of PBS (phosphate buffer solution containing 40 g of NaCl, 1 g of KCl, 5.75 g of $Na_2HPO_4$ and 1 g of $KH_2PO_4$ in 5 l of water) and further with 5% trichloroacetic acid and acetone, and dried under an infrared lamp. Then, the thymidine uptake was measured according to the conventional procedure. The measurement values shown below are averages of 3 runs of each test.

| Sample dilution | K562 | U—937 | ML—1 | THP—1 |
|---|---|---|---|---|
| | cpm | cpm | cpm | cpm |
| × 1 | — | — | 607 | 947 |
| × 2 | 948 | 799 | 470 | 2307 |
| × 4 | 1086 | 679 | 475 | 1333 |
| × 8 | 975 | 406 | 268 | 764 |
| × 16 | 881 | 377 | 359 | — |
| × 32 | 809 | 229 | 429 | — |
| FCS 10% | 449 | 535 | 680 | 1606 |
| — | 283 | 242 | 327 | 831 |

## Claims

1. Physiologically active proteinaceous substances exhibiting an activity of promoting DNA synthesis which are produced by a myelogenic leukemia cell strain and which are obtainable by culturing a myelogenic leukemia cell strain in a protein-free medium, accumulating physiologically active proteins exhibiting an activity of promoting DNA synthesis in the culture broth, and recovering the proteins from the culture broth.

2. Physiologically active proteinaceous substances according to claims 1 having the following molecular weights and isoelectric points:

| Molecular weight | Isoelectric point (pI) |
|---|---|
| 9,200 ± 500 | 8.55 ± 0.1 |
| 10,000 ± 500 | 6.9 ± 0.1 |
| 9,400 ± 500 | 8.25 ± 0.1 |
| 10,000 ± 500 | 6.7 ± 0.1 |
| 10,500 ± 500 | 5.8 ± 0.1 |
| 10,500 ± 500 | 5.6 ± 0.1 |
| 10,500 ± 500 | 6.09 ± 0.1 |
| 11,000 ± 500 | 4.7 ± 0.1 |
| 11,800 ± 500 | 4.19 ± 0.1 |
| 12,800 ± 500 | 3.54 ± 0.1 |
| 6,300 ± 500 | 8.0 ± 0.1 |
| 6,300 ± 500 | 8.4 ± 0.1 |
| 6,300 ± 500 | 8.5 ± 0.1 |
| 6,300 ± 500 | 9.0 ± 0.3 |
| 6,300 ± 500 | 9.6 ± 0.3 |

# EP 0 143 850 B1

3. A process for producing physiologically active proteinaceous substances, characterized by culturing a myelogenic leukemia cell strain in a protein-free medium, accumulating physiologically active proteins exhibiting an activity of promoting DNA synthesis in the culture broth, and recovering the proteins from the culture broth.

**Patentansprüche**

1. Physiologisch aktive proteinöse, DNA-Synthese aktivierende Stoffe, die von einer Myelom-bildenden Leukämie-Zellinie hergestellt und durch Kultivierung der Myelombildenden Leukämie-Zellinie in einem proteinfreien Medium, Ansammeln von physiologisch aktiven, DNA-Synthese aktivierenden Proteinen in einer Kulturlösung und Gewinnen der Proteine aus der Kulturlösung, erhalten werden.

2. Physiologisch aktive proteinöse Stoffe nach Anspruch 1 mit den folgenden Molekulargewichten und isoelektrischen Punkten:

| Molekulargewicht | Isoelektrischer Punkt (pI) |
|---|---|
| 9,200 ± 500 | 8.55 ± 0.1 |
| 10,000 ± 500 | 6.9 ± 0.1 |
| 9,400 ± 500 | 8.25 ± 0.1 |
| 10,000 ± 500 | 6.7 ± 0.1 |
| 10,500 ± 500 | 5.8 ± 0.1 |
| 10,500 ± 500 | 5.6 ± 0.1 |
| 10,500 ± 500 | 6.09 ± 0.1 |
| 11,000 ± 500 | 4.7 ± 0.1 |
| 11,800 ± 500 | 4.19 ± 0.1 |
| 12,800 ± 500 | 3.54 ± 0.1 |
| 6,300 ± 500 | 8.0 ± 0.1 |
| 6,300 ± 500 | 8.4 ± 0.1 |
| 6,300 ± 500 | 8.5 ± 0.1 |
| 6,300 ± 500 | 9.0 ± 0.3 |
| 6,300 ± 500 | 9.6 ± 0.3 |

3. Verfahren zur Herstellung von physiologisch aktiven proteinösen Stoffen, gekennzeichnet durch Kultivierung einer Myelom-bildenden Leukämie-Zellinie in einem proteinfreien Medium, Ansammeln von physiologisch aktiven DNA-Synthese aktivierenden Proteinen in der Kulturlösung und Gewinnen der Proteine aus der Kulturlösung.

**Revendications**

1. Substances protéinique à activité physiologique mainfestant une activité de promotion de la synthèse de l'ADN, qui sont produites par une souche de cellules de la leucémie myélogène et que l'on peut obtenir par la culture d'une souche de cellules de la leucémie myélogène dans un milieu exept de protéines, l'accumulation de protéines à activité physiologique manifestant une activité de promotion de la synthèse de l'ADN dans le bouillon de culture et la récupération des protéines du bouillon de culture.

2. Substances protéiniques à activité physiologique suivant la revendication 1, possédant les poids moléculaires et les points isoélectriques qui suivent:

11

| Poids moléculaire | Point isoélectrique (pI) |
|---|---|
| 9,200 ± 500 | 8.55 ± 0.1 |
| 10,000 ± 500 | 6.9 ± 0.1 |
| 9,400 ± 500 | 8.25 ± 0.1 |
| 10,000 ± 500 | 6.7 ± 0.1 |
| 10,500 ± 500 | 5.8 ± 0.1 |
| 10,500 ± 500 | 5.6 ± 0.1 |
| 10,500 ± 500 | 6.09 ± 0.1 |
| 11,000 ± 500 | 4.7 ± 0.1 |
| 11,800 ± 500 | 4.19 ± 0.1 |
| 12,800 ± 500 | 3.54 ± 0.1 |
| 6,300 ± 500 | 8.0 ± 0.1 |
| 6,300 ± 500 | 8.4 ± 0.1 |
| 6,300 ± 500 | 8.5 ± 0.1 |
| 6,300 ± 500 | 9.0 ± 0.3 |
| 6,300 ± 500 | 9.6 ± 0.3 |

3. Procédé de production de substance protéiniques à activité physiologique, caractérisé par la culture d'une souche de cellules de la leucémie myélogène dans un milieu exempt de protéines, l'accumulation de protéines physiologiquement actives manifestant une activité de promotion de la synthèse de l'ADN dans le bouillon de culture et la récupération des protéines du bouillon de culture.

Fig. 1

# Fig. 2

# Fig. 3

# Fig. 4

Fig. 5

## Fig. 6

# Fig. 7